# EUROPEAN PATENT APPLICATION

(11) **EP 3 517 086 A1**
(43) Date of publication of application: **31.07.2019**
(21) Application number: 18154073.3
(22) Date of filing: 30.01.2018
(51) Int. Cl.: A61F 13/42, A61F 13/00

(54) **ABSORBENT BODY**

(71) Applicant: Absorbest AB, 590 39 Kisa (SE)
(72) Inventor: ROVANIEMI, Rolf, 590 46 Rimforsa (SE)
(74) Representative: WSL Patentanwälte Partnerschaft mbB

(57) **Abstract**

According to the present invention a system is provided comprising an absorbent body having a backing layer, at least one sensor mounted on the absorbent body, wherein the sensor is arranged and located such that during operation of the system the sensor determines a parameter of the absorbent body or of a person's wound or skin , and a linking element operatively coupled to the sensor such that during operation of the system the linking element receives a signal from the sensor, wherein the linking element is arranged to transmit the signal received from the sensor to a display or another element.

## Description

The present invention relates to a system comprising an absorbent body having a backing layer, and at least one sensor mounted on the absorbent body, wherein the sensor is arranged and located such that during operation of the system the sensor determines a parameter of the absorbent body.

Examples for absorbent bodies considered in the present application are wound dressings, diapers, hygienic products and incontinence articles.

Absorbent bodies, in particular wound dressings for covering wounds, are known in various forms from the prior art.

E.g., when applied to a wound, the dressings cover the wound such that the wound is prevented from being further harmed and may heal under the dressing. Once a dressing is applied onto a wound, neither information of the status of the dressing nor information on the status of the healing are on hand, but have to be estimated based on experience. However, this has proven to be disadvantageous, especially in cases wherein unforeseen complications occur. The same lack of information occurs e.g. for a diaper worn underneath a clothing.

Thus, there is a need for gaining information on the status of an absorbent body and/or the status of a person's skin, in particular the status of the wound healing of a person's wound or skin, even when the absorbent body is used.

In WO 2012/012286 A1, systems and methods are provided for sensing fluid in a wound dressing applied to a patient and producing an electrical signal. In one instance, a galvanic cell is used as an electronic sensor. The galvanic cell is placed in the dressing and produces a voltage when the wound dressing is substantially saturated.

European patent application EP 3 034 054 A1 relates to a wound dressing comprising at least one sensor and a display, which is adapted to receive data determined by the sensor in form of an electrical signal and to display the data received.

The systems known from the prior art either provide a display fixedly mounted on an absorbent body or a display remote from the absorbent body, wherein a sensor at the absorbent body and the display are operatively coupled.

Therefore it is an object of the present invention to provide a system having more versatile applications.

At least one of the above objects is solved by a system comprising an absorbent body having a backing layer, at least one sensor mounted on the absorbent body, wherein the sensor is arranged and located such that during operation of the system the sensor determines a parameter of the absorbent body or of a person's wound or skin , and a linking element operatively coupled to the sensor such that during operation of the system the linking element receives a signal from the sensor, wherein the linking element is arranged to transmit the signal received from the sensor to a display or another element.

By use of an absorbent body according to the present invention status information about at least one parameter of the absorbent body and thus possibly of a skin on which the absorbent body is applied may be determined by the at least one sensor and displayed on the display.

In an embodiment, the absorbent body may be a wound dressing, a diaper, a hygienic product or an incontinence article.

In an embodiment of the invention the system further comprises a display operatively coupled to the linking element such that during operation of the system the display receives a signal from the linking element, wherein the display is arranged such that during operation of the system a state of the display changes depending on the signal received from the sensor, wherein the display is detachably mounted on the backing layer of the absorbent body such that it is transferable from a state wherein the display is mounted on the backing layer into a state wherein the display is remote from the backing layer. According to this embodiment of the present invention the display is detachably mounted on the backing layer of the absorbent body. Thus, in a first scenario the display during application of the absorbent body may remain on the backing layer of the absorbent body. In this case the absorbent body is used as packaged in the pouch with the display mounted on or attached to the backing layer of the absorbent body. This may in particular be advantageous for applications wherein the absorbent body is a wound dressing, e.g. if the wound dressing is applied to pressure ulcers, wherein a transparent film is placed over the entire system including the wound dressing and the display. Through the transparent film the display remains visible even when mounted on the backing layer of the wound dressing. In a second scenario the system including the wound dressing is applied to a wound using a compression bandage. Still considering an embodiment, wherein the absorbent body is a wound dressing, compression bandages are typically opaque and thus do not allow a user to look at the display if the display is mounted on the backing layer before applying the pressure bandage. However, the display according to the present invention is detachably mounted on the backing layer such that before applying the pressure bandage on the system the display may be removed from the backing layer. Then the display is no longer invisibly hidden under the pressure bandage in the applied state. Detaching the display from the backing layer may however also be advantageous for embodiments, wherein the absorbent body is a diaper, a hygienic product or an incontinence product which could be worn underneath clothing.

In the sense of the present application a state, wherein the display is detached from the backing layer of the absorbent body is considered a state, wherein the display is remote from the backing layer, i.e. not in direct or indirect contact with the backing layer, irrespective of the how far the actual distance between the display and the backing layer then is.

In an embodiment of the present invention, the system comprises a mounting means mounting the display on the backing layer. The mounting means may be a glue fixing the display on the backing layer or to an element fixed on the backing layer. In further embodiment, the mounting means is provided by breakable bridge fixing the display or a substrate supporting the display to an element, e.g. a part of the substrate, fixedly mounted on the backing layer.

In an embodiment of the present invention, the display is arranged on a substrate mounted on the backing layer. The substrate in the sense of the present application e.g. may be a plastic film, which can be glued or laminated onto the backing layer.

In an embodiment of the present disclosure, the substrate has an area being larger than an area covered by the display, wherein a part of the substrate covered by the display is detachable from a remaining part of the substrate mounted on the backing layer, which after detaching the display remains on the backing layer. This way the display and the sensor and if applicable a wire connecting the display and the sensor may be manufactured on the same substrate, e.g. by printing on the substrate. In an embodiment, a part of the substrate covered by the display to be removable from the backing layer of the absorbent body has at least one predetermined breaking point in order to be able to remove this part of the substrate and thus the display from the remaining substrate on the backing layer.

In an embodiment, there is a cutting line between the part of the substrate covered by the display and the remaining part of the substrate. It may be advantageous once the cutting line is not continuous but is interrupted by removable bridges of substrate material bridging between the part of the substrate supporting the display and the remaining part of the substrate such that by removing the bridges the display is removable form the backing layer.

In a further embodiment, the sensor is mounted on the part of the substrate remaining on the backing layer after the display is transferred into the state, wherein the display is remote from the backing layer.

In an embodiment, the linking element is at least one wire providing an operative coupling by wire between the display and the sensor. In this embodiment the at least one wire operatively connecting the sensor and the display is arranged and located such that in the state, wherein the display is mounted on the backing layer, the at least one wire is in a coiled state and further such that in the state, wherein the display is remote from the backing layer the at least one wire is in an at least partly uncoiled state. This way the wire for operatively coupling the sensor and the display can be well stored in a state, wherein the display is mounted on the backing layer. In an embodiment of the present disclosure the at least one wire is coiled on a miniaturized winder, preferably on a miniaturized and spring-biased winder.

In an embodiment of the present disclosure the at least one wire forming the linking element is mounted on a substrate detach-ably mounted on the backing layer. In a particular embodiment, this substrate supporting the wire is the same substrate or at least the same substrate material as is the substrate of the display and/or of the sensor.

In an embodiment of the present disclosure the substrate is a substrate of a plastic material, e.g. of PET (polyethylene terephthalate) or PC (polycarbonate).

As described before with respect to the substrate of the display in an embodiment the at least one wire covers part of the substrate. In an embodiment, a part of the substrate supporting the at least one wire is at least partially cut out from the rest of the substrate which remains on the backing layer. In an embodiment, the cutting lines diving the substrate supporting the wires and the substrate remaining on the backing layer are arranged such that at least one predetermined breaking point is provided mechanically connecting the part of the substrate supporting the at least one wire and at least one part of the substrate remaining on the backing layer when the display and thus at least part of the at least one wire is brought into a state remote from the backing layer.

In another embodiment, the substrate of the display and/or the substrate of the at least one wire is mounted onto the backing layer by a pressure sensitive glue.

In a further embodiment, the at least one wire providing an operative coupling between the sensor and the display in its coiled state forms two concentric spirals allowing to coil the at least one wire in a single plane and storing it with a small footprint.

In an embodiment of the present invention, the display is an electrochromic display.

In a further embodiment of the present invention, the display is based on a conducting polymer, preferably on PEDOT:PSS (Poly(3,4-ethylenedioxythiophene). PEDOT is used as coloring and counter electrode material in electrochromic displays. The electrically conducting form of PEDOT is obtained by chemical doping of the pristine conjugated polymer, and charge neutrality is then maintained by an excess amount of poly(styrene sulfonic acid) (PSS), which is a polyanion. Hence, an air-stable and electrically conducting polymer complex PEDOT:PSS is formed. PEDOT:PSS is responsive to electrochemical reduction, wherein not only the color of the material is switched, but also the electronic conductivity. The corresponding color change of the material is utilized in electrochromic displays. An electrochromic display is based on electrochemical reactions on the electrodes. One electrode is reduced and the other is oxidized. Upon changing the oxidation state, the color is changed. Usage of such a conducting polymer allows for a printable display. This display may be operable at low driving voltages of e.g. a voltage in a range from 1 V to 3 V. The display also has the advantage of being energy efficient, since it only requires an electric current during update or change of the display. To update the display a charge of only 200 nAh / cm2 may be needed. In one embodiment according to the present invention, an updating current is provided to keep the display in a high contrast mode. In an embodiment, the display is further resistant to sterilization methods used e.g. for wound dressings, like exposition to ethylene oxide gas.

In an embodiment, the at least one sensor is arranged and located to determine at least one of moisture, moisture level, pressure, temperature, or pH level as a parameter of the absorbent body or of a wound or skin to which the absorbent body is applied.

In an embodiment, the sensor is arranged and located to measure the moisture level in the wound bed, wherein the sensor is placed under the facing layer.

Furthermore, data on temperature and pH level of the wound can provide a suitable basis for conclusions on the healing process of the wound. A measurement of the pressure applied to the wound via the dressing and e.g. an additional bandage may be used to judge whether the applied pressure is too large, too small or suitable.

According to an embodiment, the sensor is located between the facing layer and the backing layer, wherein the sensor is preferably located at or integrated with the facing layer, located at or integrated with the backing layer, or located at or integrated with the absorbent core.

A suitable position of the sensor strongly depends on its nature. Temperature and leakage sensors are in some embodiments located at or integrated on or with the facing layer. A pressure sensor may be either located at or integrated on or with the facing layer or the backing layer.

A moisture-detecting sensor is placed wherever a local occurrence or level of moisture is to be determined. Leakage in general occurs at the facing layer, which results in a need to have a sensor located peripherally between the facing layer and the absorbent core. For detection of saturation, the sensor may instead be placed at the backing layer to sense, when the fluid wets through the absorbent core. To be able to detect pattern of local saturation, there is a need to have sensors spread over larger areas, e.g. in a net or matrix shaped pattern. In one embodiment, moisture sensors are distributed according to a 3D matrix pattern over the absorbent body, thus providing information on the 3D distribution of moisture within the absorbent body.

Pressure and temperature may be measured based on determining changes in resistance. Moisture may be measured by a potential generated due to the contact between two electrodes of the sensor and an electrolytic fluid, e.g. urine, wound fluid or wound exudate.

A measurement of a moisture level is intended to indicate, when it is time to replace the absorbent body. The switching time of the corresponding sensors and display may be set on the order of minutes, since the wetting process of an absorbent core, e.g. with superabsorbent particles, is rather slow, i.e. occurs on similar time scales. The display should be capable to show a corresponding message in a range from about 1/2 day to 7 days, which is the usual period during which a absorbent body is applied to a person's skin or wound before disposal. It may be beneficial to have an icon, e.g. on the display, that is activated and lights up as soon as the absorbent body is applied and/or the sensor system/display is activated in order to indicate that the same are working.

In an embodiment of the invention, at least one sensor determines the absolute temperature in order to fulfil the need to indicate and report changes in temperature. In one embodiment, the sensor is adapted for a temperature range of 32 °C to 43 °C. For displaying the temperature in both °C as well as °F a switch device for altering between the values may be provided. Switching time of the sensor and/or display may be on the order of minutes, since the change in temperature is a comparably slow process. In one embodiment, a sensor for determining the temperature is located in the center of the absorbent body close to the surface of the wound. In one embodiment, a temperature sensor is placed on or under the facing layer in order to determine the temperature of the wound as directly as possible.

In one embodiment of the present invention, thermistors and thermocouples are used as temperature sensors. Thermistors may be NTC (negative temperature coefficient) as well as PTC (positive temperature coefficient). In order to apply those sensors to the absorbent body preferably printable ink with thermistors may be used, e.g. nano silicon based ink with NTC thermistors. In one embodiment, a printable ink for an inkjet printer or for a screen printer is used to create a sensitive thermistor. The first layer thereof consists of conductors made of silver and the second layer is made of nickel oxide (NiO) that act as a thermistor due to the semiconducting behavior that changes, when exposed to variations in the temperature. In another embodiment, thermistors are applied that can be surface mounted (SMD) using conducting glue. The thermistor may beneficially be encapsulated in glass for higher accuracy and lower moisture sensitivity.

In particular in an embodiment, wherein the absorbent body is a wound dressing, a further parameter of interest is the absolute pressure, in particular the pressure generated by applying the dressing onto a wound. Therefore, in an embodiment of the present invention a pressure-determining sensor is provided, which is e.g. adapted for a pressure range of 18 mmHg to 70 mmHg with a resolution of 5 mmHg, corresponding to a range of 2.4 kPa to 9.3 kPa with a resolution of 0.7 kPa or a range of 0.4 psi to 1.4 psi. The pressure measured in mmHg may e.g. be visualized in steps of 5 mmHg via the display. This enables simpler drive electronics and a smaller number of display segments. Switching time for sensor and display may beneficially be on the order of seconds, e.g. one second, in order to provide real time information on pressure during fixation of the wound dressing on the wound. In one embodiment, one sensor is located close to the backing of the dressing, i.e. close to, on or under the backing layer.

Different types of pressure sensors may be used for a wound according to the present invention, e.g. piezo resistors, percolations resistors and quantum tunneling composites (QTC). In one embodiment, QTC materials, which change their electrical resistance under pressure, are used in form of an in screen printable ink. QTC materials are based on irregular particles that come close to each other and provide tunneling of electrons between the particles. The materials used to create the particles are e.g. based on Nickel, Copper and Gold. The more particles come close together, the lower the resistance is.

In an embodiment, the at least one sensor is formed by a galvanic cell, wherein the sensor comprises two electrodes, i.e. an anode and a cathode, which are adapted to be activated by a fluid. Thus, in order to generate voltage, the electrodes have to be exposed to the fluid acting as an electrolyte. E.g. wound fluid in general consist to 0.9% of NaCl and is therefore suitable as an electrolytic fluid for a galvanic element. A galvanic element in terms of the present invention is every combination of at least two different electrodes and an electrolyte. In another embodiment of the present invention, the galvanic cell is formed by a sensor comprising two different electrodes of different metals.

The sensor when in contact with the fluid becomes a battery and in turn provides a voltage to drive a change of the display. Thus, the display is updated by the sensor to indicate that it is time to change the absorbent body, when fluid connects the two electrodes of the sensor generating a potential and a corresponding voltage signal is transferred to the display.

Three factors define the capability of a sensor to function as a galvanic cell, which are the electrode materials, capacity and the internal resistance. The choice of the electrode material determines the theoretical voltage that can be generated. The internal resistance influences the practical output voltage generated from the sensor. The capacity is determined by the amount of active material used in the electrode, which needs to contain enough charge carriers to update the display.

In an embodiment the electrodes of the sensor forming a galvanic cell are provided on a substrate, wherein in an embodiment the substrate supporting the electrodes of the sensor is the same substrate as the substrate supporting the display and/or the at least one wire forming the linking element. In an embodiment the material of the substrate is the same material as the material of the display and/or the at least one wire.

In an embodiment, a cutting line is provided dividing the substrate between the two electrodes of the sensor in order to avoid condensation on the substrate.

According to another embodiment, a protrusion is provided on the substrate protruding from the substrate between the two electrodes of the sensor. This way a bridge separating the two electrodes is provided on the substrate.

In yet another embodiment, at least one of the electrodes of the sensor is coated with a coating material leading to a delayed response of the sensor. In an embodiment of the present invention the coating is dissoluble in the exudate such that after a time delay it exposes the electrode coated.

According to one embodiment of the invention, the sensor is printed by using a mixture of ink with metal oxides, wherein for cathode and anode different types of oxides are used.

In an embodiment, a material for the anode may comprise zinc. In an embodiment, the zinc is provided as a coating on carbon or blended with carbon at different ratios. The zinc particles may be mixed with carbon in order to obtain a uniform conductivity in the electrode.

In an embodiment, the cathode may comprise MnO₂ blended with carbon at different ratios to achieve a higher conductivity.

In another embodiment, the cathode may comprise PE-DOT:PSS.

In yet another embodiment, the cathode may comprise silver oxide. The silver oxide may preferably be divalent (AgO) or monovalent (Ag₂O) silver oxide. The conductivity of the monovalent silver oxide is however rather poor. To increase the conductivity of the silver oxide, in an embodiment the silver oxide may be mixed with carbon powder.

In yet another embodiment, a Fe/AgO combination is used, providing high reliability, long life and better durability. Iron and silver oxide in an embodiment, may be mixed in a carbon ink to achieve functionalized electrodes.

Furthermore, in one embodiment a bio fuel cell is used, which is triggered by the fluid. This technology is based on enzyme reactions or bacteria that mimics the interactions found in nature.

The electrodes in the at least one sensor in an embodiment may be based on silver, carbon, or conducting polymers, which may be patterned using common printing technologies.

In an embodiment, the electrodes are flat and have a preferably circular, elliptical, rectangular or square shape. In another embodiment, the electrodes are elongated and form closed loops with a preferably circular, elliptical, rectangular or square shape.

Taking into account the general propagation path of fluid in the absorbent body, the at least one sensor may be formed and placed such that it is exposed to moisture, when a certain level of saturation is reached. Alternatively or additionally, a sensor may be placed such that the fluid has to come in contact with the same, when the fluid is going to leak out of the absorbent body.

According to an embodiment, the absorbent body comprises at least two sensors, the electrodes of which are arranged concentrically within in a common plane and/or on parallel planes. The sensors will trigger as soon as the liquid reaches a portion of the electrodes. Thus, activation of the sensors one after another provides information on the dynamical progress of the fluid within the absorbent body.

In an embodiment comprising at least two sensors, the electrodes are elongated, straight and extend parallel to each other within in a common plane and/or on parallel planes.

In an embodiment, a plurality of sensors are positioned according to a 2D and/or a 3D matrix. Based on the data gained by those sensor are 2D or 3D pictures of the moisture distribution and its progress within the absorbent body may be provided on the display. According to one embodiment, the absorbent core is formed by at least three absorbent sub-layers, wherein at least two sensors are arranged between different sub-layers. Thereby a complex 3D distribution of sensors within the absorbent body is implemented.

The system according to a further embodiment in addition to the linking element in the form of at least one wire may comprise a wireless transmitter operatively coupled to the display and/or to the sensor to receive a signal from the sensor and/or the display, wherein the wireless transmitter is arranged to provide a wireless to a communication equipment in order to transmit a signal to the communication equipment. This embodiment enables read out of the measurement result obtained by the sensor e.g. by a mobile device like a smart phone or a tablet. In an embodiment of the present invention, the wireless transmitter is a NFC chip or a Bluetooth device.

According to an alternative embodiment the linking element is a wireless transmitter operatively coupled to the sensor in order to wirelessly transmit the signal received from the sensor to a display. In this embodiment the display typically will not be part of the system, but will be part e.g. of a mobile device like a phone or a laptop computer. In this embodiment there is no wired communication between the sensor and a display. There are numerous ways to provide a mobile communication between the system comprising the absorbent body, the sensor and the wireless transmitter and a further device. In an embodiment the transmitter is an active device in that the system comprises an additional power supply, like a rechargeable battery, enabling an active transmission of signals from the transmitter to the other device. In the alternative embodiment, the transmitter is a passive device in that it has to be powered from the outside, e.g. inductively. In this embodiment the system may not have its own power supply. An example for a passive transmitter is an RFID tag, e.g. with an inductive power supply. In an embodiment the RFID tag will be connected to a sensor in the form of a galvanic cell.
A wound dressing in terms of the present application refers to any wound care article for covering a wound such that the wound is prevented from being further harmed and may heal under the dressing. A wound dressing may e.g. be a sterile pad, a compress, a bandage, an adhesive bandage, like a Band-Aid®, or a plaster.

The absorbent body according to the present invention comprises a backing layer. When the absorbent body is applied to a person's skin, the backing layer is not in direct contact with the skin. The backing layer may serve as a cover of the absorbent body. Now considering a case, wherein the absorbent body is a wound dressing, when the wound dressing is applied to a person's skin, the backing layer is not in direct contact with the skin. The backing layer may serve as a cover of the wound dressing.

In an embodiment, the wound dressing is designed to be in direct contact with the wound. In one embodiment, according to the present invention, the wound dressing is adapted for absorbing wound exudate discharged from a wound.

In an embodiment according to the present invention, the wound dressing is a self-adhesive bandage comprising a backing layer, an absorbent pad and an adhesive film. In an embodiment, the absorbent pad is located underneath the backing layer, wherein the absorbent pad is intended to come into direct contact with a wound and is suitable to absorb exudate from the wound. The backing layer prevents direct contact between the absorbent pad and e.g. a patient's clothing. For example a self-adhesive film may be applied to the bottom side of the backing layer or as a backing layer, which laterally extends beyond the absorbent pad. Those sections extending beyond the absorbent pad may be attached to a patient's skin. In a particular embodiment, the wound dressing forms a plaster.

In one embodiment, the absorbent body comprises an absorbent core, a facing layer, and a backing layer, wherein the absorbent core is located between the facing layer and the backing layer.

A location on the backing layer hereinbefore or hereinafter refers to being placed/located on an exterior surface of the backing layer, i.e. on an exterior surface of the absorbent body. A location under the backing layer refers to being placed/located on an interior surface of the backing layer, i.e. inside the absorbent body. Placed/located on the facing layer refers to being placed/located on an interior surface of the facing layer, i.e. inside the absorbent body. Placed/located under the facing layer refers to being placed/located on an exterior surface of the facing layer, i.e. on an exterior surface of the absorbent body, intended to make direct contact with the wound.

Fluid discharged from a person is absorbed by the absorbent core of the absorbent body through the facing layer. Once the core is saturated, the absorbent body has to be replaced by a new one. Furthermore, when becoming saturated the absorbent body may tend to provide leakage of fluid. A problematic behavior of an absorbent core, particularly of an absorbent core comprising a superabsorbent, is that the superabsorbent can create a "gel blocking" resulting in blocking of the lateral flow, which negatively influences the filling behavior of the absorbent core.

By a absorbent body according to the present invention indication may be provided in time before saturation or leakage of the absorbent body occurs. In addition, a high local moisture content, which can be disadvantageous for a wound's healing process, may be determined based on the data shown by the display. Thus, it may be easily judged on basis of suitable indicators, when it is time to replace an absorbent body. In particular, even unforeseen complications like "gel blocking" may be resolved in time by replacing the absorbent body.

Absorbent bodies are in general intended as disposable items for reasons of hygiene. The absorbent body comprises a layered structure in an embodiment having at least a facing layer, an absorbent core and a backing layer. A wide range of suitable structures and materials may be used for the absorbent body.

In an embodiment, the absorbent core may be any structure suitable to absorb exudate from a wound. The material of the absorbent core in an embodiment may comprise any one of a group consisting of cellulose, regenerated cellulose, in particular cellulose fluff or regenerated cellulose fluff, air-laid cellulose or air-laid regenerated cellulose, tissue paper, a non-woven, a textile fabric, a foam, an alginate, ALT, and a hydrocolloid or a combination thereof. In one embodiment, the absorbent core is produced as a spunlaced web material of 100% pure cellulose or 100% regenerated cellulose. In another embodiment the absorbent core comprises a mixture of pure cellulose or regenerated cellulose and synthetic fibers. In yet another embodiment the absorbent core comprises a non-woven tamponade or pad containing sodium carboxymethyl cellulose and regenerated cellulose, as it is commercially available under the trade name aquacel® from ConvaTec (Germany) GmbH of Munich, Germany.

In one embodiment of the present invention, the absorbent body comprises an absorbent pad, which is suitable to absorb blood from a wound. The material of the absorbent pad in an embodiment may comprise any one and any combination of the materials listed above as materials of an absorbent core.

In an embodiment of the invention, such a structure of the absorbent core may be used as a carrier layer to accommodate or carry an absorbent substance, in particular a superabsorbent substance.

Superabsorbent substances in the sense of the present application are materials being able to absorb and retain large volumes of water in aqueous solutions. Superabsorbent substances falling into this category are for example modified starch, polymerized polyvinyl alcohol (PVA) and polyethylene oxide (PEO) which are all hydrophilic and have a high affinity to water. When chemically or physically cross-linked, these polymers are water-swellable but not water-soluble. The aforementioned superabsorbent substances have been known for a long time.

In a particular embodiment of the present invention, the superabsorbent substance is a superabsorbent polymer (SAP), in particular in the form of (granular) particles or fibers. In an embodiment, such a SAP is made from polymerization of acrylic acids blended with sodium hydroxide in the presence of an initiated form poly-acrylic acid sodium salt (sometimes referred to a sodium polyacrylate).

In a further embodiment, the absorbent core containing SAP comprises a carrier layer, wherein the superabsorbent polymer is dispersed in the carrier layer. In an embodiment, the carrier layer in particular may comprise a material selected of a group consisting of tissue paper, a spunlaced polymer, a non-woven fabric, fluff/cellulose, regenerated cellulose such as viscose, foam based on different chemistry as polyurethane, alginate, hydrocolloid, carboxymethyl cellulose (CMC) and its derivate and cotton.

In an embodiment, the absorbent core containing SAP comprises at least two carrier layers, which together with the SAP form the absorbent core. For manufacturing, the SAP is dispersed on the first layer, then the second layer is put on top and the two layers are consolidated providing a matrix carrying the SAP between the two layers.

In an embodiment, the absorbent core comprises a carrier layer made of a spunlaced polymer as a non-woven fabric and a granular or fibrous SAP. For manufacturing, the SAP is in a first step, preferably uniformly, dispersed on a first sheet or layer of the spunlaced nonwoven. In a second step, a second sheet or layer of the spunlaced nonwoven is put on top of the first sheet, such that the SAP is located between the two sheets or layers. Then the SAP is integrated in both layers by applying pressure to this sandwich structure provided. By applying pressure, the two layers of spunlaced polymer are consolidated and the SAP to some extent fills up voids in the spunlaced material. The laminate formed this way is soft and looks like a single uniform layer of material.

A non-woven fabric in the sense of the present application is a material made of at least one layer of fibers that have been formed to a web and consolidated in a next step. In particular, consolidation of the non-woven fabric may be achieved by friction and/or cohesion and/or adhesion, for example by needling, felting, spun-lacing, melting or heat embossing.

If compared to tissue paper a material will be considered a non-woven fabric in the sense of the present application once more than 50 % of the mass of its fiber components consist of fibers having a ratio of their length to their diameter of more than 300. Alternatively, the material will be considered a non-woven fabric in the sense of the present application if this condition is not fulfilled, but if more than 30 % of the mass of its fiber components consist of fibers having a ratio of their length to their diameter of more than 300 and its density is lower than 0.4 g / cm3. This is deemed to be equal to EM 29 092.

While an absorbent core as described above may be advantageous, it is not excluded to design absorbent cores using different material combinations.

An absorbent core, in particular an absorbent core having a superabsorbent substance that in the following text may also be denoted as a superabsorbent core, extracts and stores liquid stemming from a person's body.

In order to avoid direct contact between the absorbent core and the skin, the absorbent body further comprises a facing layer.

In order to avoid direct contact between the absorbent core and e.g. a patient's clothing, the absorbent body further comprises a backing layer.

In an embodiment, the facing layer comprises a material selected of a group consisting of a non-woven fabric, e.g. containing polyethylene (PE), polyethylene terephthalate (PET), polypropylene (PP), polyamide or polytetrafluoroethylene (PTFE), a perforated sheet, e.g. containing polyethylene (PE), polyethylene terephthalate (PET), polypropylene (PP), poly-amide or polytetrafluoroethylene (PTFE), a perforated sheet laminated on a non-woven fabric, e.g. containing polyethylene (PE), polyethylene terephthalate (PET), polypropylene (PP), polyamide or polytetrafluoroethylene (PTFE), a fine net or screen, e.g. containing polyethylene (PE), polyethylene terephthalate (PET), polypropylene (PP), polyamide or polytetrafluoroethylene (PTFE), a perforated foam or sheet comprising polyurethane, a perforated material based on silicone or a foam with open cells based on polyurethane or silicone or a combination thereof.

In an embodiment, the facing layer comprises a perforated sheet or film, wherein perforations in the facing layer form a three-dimensional structure in order to reduce the sticking surface between the facing layer and the wound surface and to enhance the contact with the wound.

In an embodiment, the facing layer comprises a non-woven fabric consisting of synthetic and/or cellulose fibers. In an embodiment, the fibers of a non-woven fabric forming a facing layer are reoriented such that they predominantly extend in a direction perpendicular to the extension of the facing layer. Such a reorientation of the fibers in the non-woven fabric is achieved by orienting the fibers during the fabrication process, in particular during spun-lacing, needling or electrostatic processing.

In an embodiment of the invention, the facing layer comprises a density in a range from 0.1 g / cm3 to 0.6 g / cm3.

In a further embodiment, the facing layer made of a nonwoven in its unwetted state comprises an area weight (also denoted as the gram weight, basis weight or grammage) in a range from 8 gsm (g / m2) to 50 gsm (g / m2), preferably in a range from 12 gsm (g / m2) to 30 gsm (g / m2).

It is further useful if in an embodiment the facing layer comprises a hydrophobic or hydrophilic surface. This is in particular applicable once the facing layer is brought into direct contact with the wound. Therefore, the facing layer in an embodiment may be coated by non-sticking material, e.g. silicone. In an embodiment, the non-sticking material on the facing layer may be structured to form a pattern with holes on the facing layer.

While it may be that the facing layer is brought into direct contact with the skin (it may then be denoted the contact layer), there may be an embodiment, wherein the absorbent body comprises one or more further layers between the facing layer and the wound. Depending on its functionality in the absorbent body, a person skilled in the art will choose the material of the facing layer in order to fulfil such functionality.

In an embodiment of the present invention, the backing layer serves as a clothing protection. In an embodiment, the backing layer thus is advantageously made of a breathable, non-woven fabric or a breathable film enabling breathing of the wound, but preventing fluids from exiting the absorbent body and contaminating a patient's clothing. In a further embodiment the backing layer, in particular when made of a non-woven fabric, is hydrophobic.

In an embodiment of the invention, the backing layer is a hydrophobic nonwoven based on polypropylene with a hydro head in a range from 40 cm H₂O to 120 cm H₂O, preferably in a range from 50 cm H₂O to 80 cm H₂O. A backing fulfilling this requirement on the one hand provides a good protection of a person's clothing while on the other hand avoiding bacteria to enter into the absorbent body. The hydro head in the sense of the present application is the height of a vertical water-column standing on and above the surface of the material and which the material can stand against before the water passes through the material to the other side. The numbers given for the hydro head are measured according to ISO 811:1981.

In an embodiment of the invention, the absorbent body comprises a backing layer, e.g. a BTBS (breathable textile back sheet), folded such that it prevents side leakage. An effective prevention of leakage can be achieved by a tubular arrangement, wherein a circumferential portion of the backing layer is folded around the edges of the absorbent core such that it extends at least partially below the absorbent core and overlaps the facing layer. The overlapping portion in direct contact with the facing layer is fixed onto the same, e.g. by gluing. Thereby a leakage of absorbed fluid at the side portions of the absorbent body may be effectively prohibited by a backing layer, especially a BTBS backing layer.

Further advantages, features and applications of the present invention will become apparent from the following description of embodiments and the corresponding figures attached.
- Figure 1: is a schematic bottom view of a system according to an embodiment of the present invention.
- Figure 2: is a schematic bottom view of a system according to a second embodiment of the present invention.
- Figure 3: is a schematic bottom view of a system according to yet another embodiment of the present invention.

In the figures identical elements have been denoted by identical reference numbers. The figures represent schematic views only and are not to scale.

In the embodiments described hereinafter in detail the absorbent body is a wound dressing 2, wherein the linking element is provided by at least one wire 8, 9.

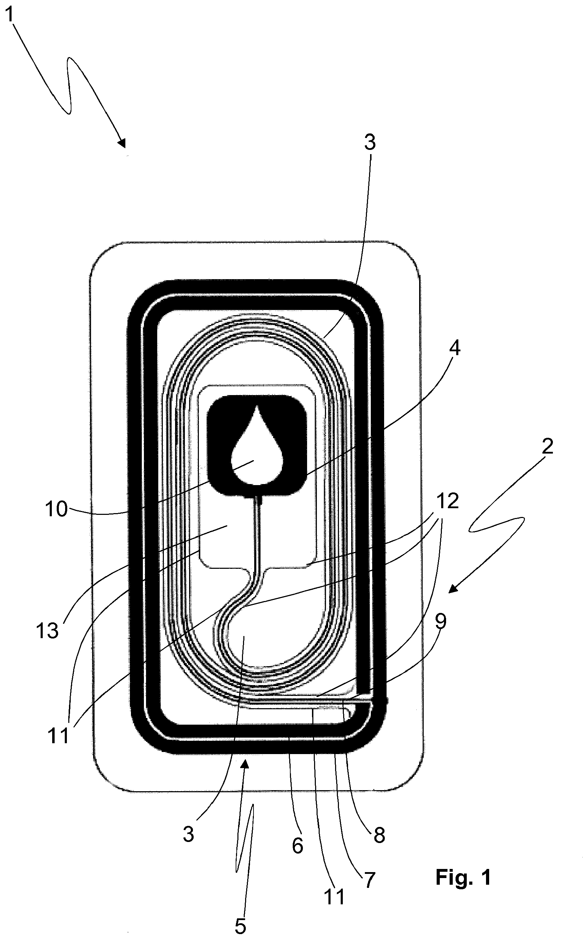

Figure 1 is a schematic bottom view of the backing layer 3 of a wound dressing 2 forming part of a system 1 according to an embodiment of the present invention.

The wound dressing 2 forming the basis of all embodiments of the system 1, 1', 1" according to the present invention as described with reference to figures 1 to 3 is a superabsorbent wound dressing 2. Further to the wound dressing 2 each of the systems 1, 1', 1" depicted in figures 1 to 3 comprises a display 4, 4'.

In the figures the displays 4, 4' are shown in a state mounted on the backing layer 3 of the wound dressing 2. The state shown in the figures corresponds to the state in which the system is taken out of the pouch.

If the system 1, 1', 1" is applied to a pressure ulcer or any other ulcer that does not require to be covered with an opaque bandage, the wound dressing 2 is applied to the patient's wound and then the entire system including the dressing 2 and the display 4 is covered by a pressure adhesive transparent film. Due to the transparency of the film the display 4 remains visible without detaching the display 4 from the backing layer 3.

However, the system 1, 1', 1" according to the present invention can also be used once the system 1, 1', 1" is attached to a patient's wound using an opaque pressure bandage. In order to be able to still view the display 4 once the wound dressing is covered by an opaque bandage the display 4 is detachably mounted on the backing layer 3.

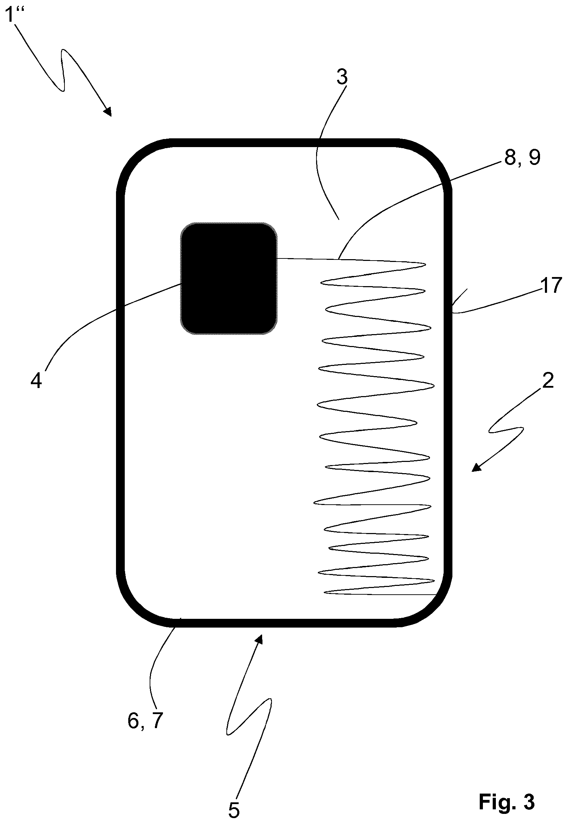

In all three embodiments according to figures 1 to 3 the sensor 5 of the system 1, 1', 1" is provided by a galvanic cell comprising two electrodes 6, 7 each. In the schematic representation of figure 3 the two electrodes 6, 7 are simplified to a single black line. In the embodiments depicted the electrodes 6, 7 enclose the display 4, 4' when mounted on the backing layer 3 as well as the wires connecting the display 4, 4'. The electrodes 6, 7 of the sensor 5 form the electrodes of a galvanic element.

The display 4, the wires connecting the display 4 and the sensor 5 as well as the sensor 5 are manufactured on a plastic substrate which in turn is laminated partly onto the backing layer 3 of the wound dressing 2. The electrodes 6, 7 are structured on the surface of the substrate facing towards the backing layer 3 in order to be in direct contact with the backing layer 3. When wound exudate spreads within the super absorbent core (not shown in the figures) of the wound dressing 2 it will finally reach the two electrodes 6, 7 of the sensor 5 and establish an electric contact between the two electrodes 6, 7. Thus, the galvanic element constituted by the two electrodes of which the first electrode forms an anode 6 and the second electrode forms a cathode 7 will be activated by the electrolytic wound exudate and a corresponding potential is generated between the two electrodes 6, 7. The resulting voltage signal is applied to the two wires 8, 9 and via the wires 8, 9 to the display 4. Thus, the resulting voltage signal will update the display 4. The display 4 in the embodiments depicted shows a schematic representation of a drop of liquid 10 which changes its color upon application of a certain threshold voltage. The larger the closed loop formed by the two electrodes 6, 7 of the sensor 5 is, the larger the saturation level of the wound dressing 2 is, which has to be reached before the wound exudate comes to into contact with the electrodes 6, 7. The sensor 5 is preferably located such that the display 4 indicates a saturation degree sufficiently below 100% to indicate in time that it is necessary to change the wound dressing too.

In order for the display 4 to be detachable from the backing layer 3 of the wound dressing 2 the substrate carrying the electric components 4, 6, 7, 8, 9 is cut before laminating parts of the substrate onto the backing layer 3 of the wound dressing 2. In the embodiment shown in figure 1 the wires 8, 9 connecting the sensor 5 and the display 4 are wound inwardly starting from the electrodes 6, 7 of the sensor 5 in two turns around the display 4 in order to end at the display 4. The substrate is cut parallel to the path of the wires 8, 9, such that the substrate carrying the wires 8, 9 can be removed from the rest of the substrate. The cuts are denoted by reference numbers 11, 12 in figure 1. Finally also the part of the substrate 13 carrying the display 4 is cut out such that this part of the substrate 13 can also be removed from the rest of the substrate. The cutting lines 11, 12 are positioned such that the coiled wires 8, 9 on the substrate can be removed from the backing layer 3 as well as this part 13 of the substrate carrying the display 4 and the wires 8, 9 can be uncoiled in order to position the display 4 remote from the backing layer 3.

It has to be pointed out that the substrate in those regions carrying the wires 8, 9 and the display 4 is not laminated or otherwise mounted onto the backing layer 3.

Still, in order to avoid that the display 4 and the wires 8, 9 or their respective parts of the substrate will fall off the backing layer 3 when the system 1 is unwrapped from the pouch at least the part 13 of the substrate carrying the display 4 is not completely cut out, but at least one land connecting the part of the substrate 13 carrying the display 4 and the neighboring part of the substrate remains. This land can easily be destroyed or torn apart when the part 13 of the substrate carrying the display 4 is removed from the backing layer 3.

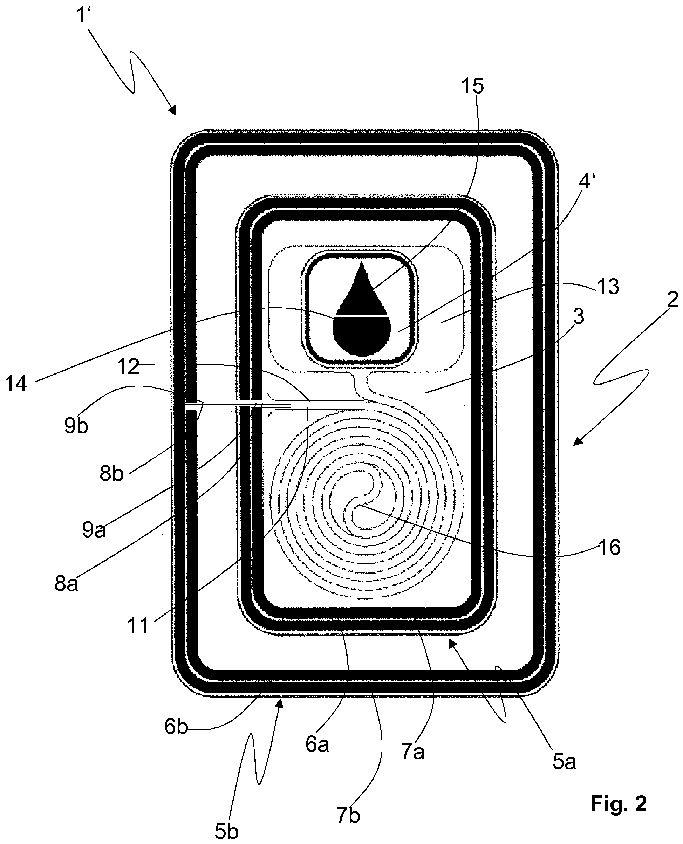

The embodiment of the system 1' of figure 2 differs from the embodiment of figure 1 with respect to two aspects. First of all the display 4' is modified when compared to the display 4 of figure 1. The display 4' shows a schematic representation of a droplet of liquid comprising two segments 14, 15. The two segments 14, 15of the display 4' are connected to two different sensors 5a, 5b each comprising two electrodes 6a, 7a and 6b, 7b. Exudate being absorbed by the wound dressing 2 will spread from a point or area enclosed by the inner sensor 5a radially outwardly. It will first get in contact with the electrodes 6a, 7a of the first sensor 5a and activate the galvanic element formed by the electrodes 6a, 7a. When absorbing further fluid, the fluid will finally reach the electrodes 6b, 7b of the second sensor 5b and activate the galvanic cell formed by those electrodes 6b, 7b. Consequently, the display 4' may indicate two different states of the wound dressing 2 during absorption of wound exudate in its absorbent core.

Furthermore the wires 8, 9 have been coiled up in a double spiral, wherein starting from the electrodes 6, 7 of the sensor 5 the wires 8, 9 are coiled inwardly to reach a' center 16 of the spiral and then the wires are coiled outwardly to finally reach the part 13 of the substrate carrying the display 4'. This design of the coiling allows to provide a longer connection by wire between the sensor 5 and the display 4'.

Figure 3 schematically depicts a further design of the winding of the two wires 8, 9 between the electrodes 6, 7 of the sensor 5 and the display 4. The wires 8, 9 are coiled on a zigzag path along the long edge 17 of the electrodes 6, 7 of the sensor 5.

For purposes of original disclosure it is pointed out that all features which are apparent for a person skilled in the art from the present description, the figures and the claims, even if they have only been described with further features, could be combined on their own or together with all the combinations of the features disclosed herein, if not excluded explicitly or technically impossible. A comprehensive explicit description of all possible combinations of features is only omitted in order to provide readability of the description.

### List of reference numbers

- 1, 1', 1": system
- 2: wound dressing
- 3: backing layer
- 4, 4': display
- 5: sensor
- 5a: first sensor
- 5b: second sensor
- 6, 6a, 6b: electrode / anode
- 7, 7a, 7b: electrode / cathode
- 8: wire
- 9: wire
- 10: display in the form of a liquid drop
- 11: cutting line
- 12: cutting line
- 13: substrate
- 14: display segment
- 15: display segment
- 16: center
- 17: edge

## Claims

1. A system (1, 1', 1") comprising
an absorbent body (2) having a backing layer (3),
at least one sensor (5) mounted on the absorbent body (2), wherein the sensor (5) is arranged and located such that during operation of the system (1, 1', 1") the sensor (5) determines a parameter of the absorbent body (2) or of a person's wound or skin, and
a linking element (8, 9) operatively coupled to the sensor (5) such that during operation of the system (1, 1', 1 ") the linking element (8, 9) receives a signal from the sensor, wherein the linking element (8, 9) is arranged to transmit the signal received from the sensor (5) to a display (4, 4') or another element.

2. The system (1, 1', 1 ") according to the previous claim, wherein the system (1, 1', 1 ") further comprises a display (4, 4') operatively coupled to the linking element (8, 9) such that during operation of the system (1, 1', 1 ") the display (4, 4') receives a signal from the linking element (), wherein the display (4, 4') is arranged such that during operation of the system (1, 1', 1 ") a state of the display (4, 4') changes depending on the signal received from the sensor (5), and wherein the display (4, 4') is detachably mounted on the backing layer (3) of the absorbent body (2) such that it is transferable from a state wherein the display (4, 4') is mounted on the backing layer (3) into a state wherein the display (4, 4') is remote from the backing layer (3).

3. The system (1, 1', 1") according to the previous claims, wherein the display (4, 4') is arranged on a substrate (13) mounted on the backing layer (3).

4. The system (1, 1', 1 ") according to the previous claim, wherein the substrate (13) has an area being larger than an area covered by the display (4, 4'), wherein a part of the substrate (13) covered by the display (4, 4') is detachable from a remaining part of the substrate (13) mounted on the backing layer (3) which after detaching the display (4, 4') remains on the backing layer (3).

5. The system (1, 1', 1 ") according to the previous claim, wherein the sensor (5) is mounted on the part of the substrate (13) remaining on the backing layer (3) after the display (4, 4') is transferred into the state, wherein the display (4, 4') is remote from the backing layer (3).

6. The system (1, 1', 1") according to claim 4 or 5, wherein there is a cutting line between the part of the substrate (13) covered by the display (4, 4') and the remaining part of the substrate (13).

7. The system (1, 1', 1") according to any one of the previous claims, wherein the linking element is a wireless transmitter, preferably a RFID transponder, in order to couple the sensor (5) wirelessly to a display (4, 4').

8. The system (1, 1', 1 ") according to any one of claims 1 to 6, wherein the linking element is at least one wire (8, 9) to couple the sensor (5) to a display (4, 4') by wire (8, 9).

9. The system (1, 1', 1 ") according to the previous claim, wherein the at least one wire (8, 9) operatively coupling the sensor (5) and the display (4, 4') is arranged and located such that in the state wherein the display (4, 4') is mounted on the backing layer (3) the at least one wire (8, 9) is in a coiled state and such that in the state wherein the display (4, 4') is remote from the backing layer (3) the at least one wire (8, 9) is in an at least partly uncoiled state.

10. The system (1, 1', 1 ") according to the previous claim, wherein the at least one wire (8, 9) is mounted on a substrate (13) detachably mounted on the backing layer (3).

11. The system (1, 1', 1 ") according to claim 9 or 10, wherein the at least one wire (8, 9) in its coiled state forms two concentric spirals.

12. The system (1, 1', 1 ") according to any one of the previous claims, wherein the sensor (5) is a galvanic cell, wherein the sensor (5) comprises two electrodes (6, 7), which are arranged to be activated by a fluid.

13. The system (1, 1', 1 ") according to any one of the previous claims, wherein the absorbent body (2) further comprises an absorbent core and a facing layer wherein the absorbent core is located between the facing layer and the backing layer (3).

14. The system (1, 1', 1") according to the previous claim, wherein the absorbent body (2) comprises a superabsorbent substance, and wherein the superabsorbent substance is preferably located in the absorbent core.

15. The system (1, 1', 1") according to any one of the previous claims, wherein the absorbent body (2) is a wound dressing (2), a diaper or an incontinence article.
